# EUROPÄISCHE PATENTSCHRIFT

(11) **EP 1 962 800 B1**
(45) Veröffentlichungstag und Bekanntmachung des Hinweises auf die Patenterteilung: **14.10.2015**
(21) Anmeldenummer: 06829650.8
(22) Anmeldetag: 15.12.2006
(51) Int. Cl.: A61L 15/46, A61L 15/60, A61L 15/18, A61K 33/38

(54) **ANTIMIKROBIELLE WUNDKONTAKTSCHICHT**
ANTIMICROBIAL WOUND CONTACT LAYER
COUCHE ANTIMICROBIENNE EN CONTACT AVEC LA PLAIE

(30) Priorität: 17.12.2005 DE 102005060461
(43) Veröffentlichungstag der Anmeldung: 03.09.2008
(73) Patentinhaber: Paul Hartmann AG, 89522 Heidenheim (DE)
(72) Erfinder: EFFING, Jochem, 65779 Kelkheim-Fischbach (DE)
(86) Internationale Anmeldenummer: PCT/EP2006/012100
(87) Internationale Veröffentlichungsnummer: WO 2007/068490

(56) Entgegenhaltungen:
- EP-A2- 1 159 972
- WO-A-01/60599
- DE-A1-102004 031 955
- US-A- 4 503 034
- US-A1- 2004 049 145

## Beschreibung

Die vorliegende Erfindung betrifft eine antimikrobielle Wundkontaktschicht sowie deren Verwendung zur Wundbehandlung.

Eine Vielzahl an Salben oder anderen Zusammensetzungen sind in den vergangenen Dekaden zur Behandlung am Menschen bekannt und eingesetzt geworden. Diese Salben sind meist halbfeste Zusammensetzungen, die zur Anwendung auf gesunder Haut oder einigen Schleimhäuten wie beispielsweise auf den Augen bestimmt sind. Meist soll durch diese Salben oder Zubereitungen eine lokale Wirkung ausgeübt, Wirkstoffe perkutan verabreicht oder eine erweichende oder schützende Wirkung auf die Haut ausgeübt werden.

Darüber hinaus sind auch zahlreiche Salben zur Wundversorgung bekannt. So beschreibt beispielsweise die EP 621 031 eine Wundsalbe, die als Gel formuliert ist und mindestens ein gelbildendes Polysaccharid und Hexyleneglycol umfasst. Als gelbildendes Polysaccharid soll insbesondere Carboxymethylcellulose oder Natriumalginat eingesetzt werden. Diese Zusammensetzung soll eine antimikrobielle Wirkung aufweisen und nicht toxisch in Bezug auf Fibroplasten sein.

Des Weiteren wird mit der EP 107 526 eine Paste zum Schutz der Haut beispielsweise bei der Wundbehandlung oder der Stomaversorgung beschrieben, die als Gel formuliert Polyvinylpyrrolidon, Carboxymethylcellulose, Alginat, Wasser, ein Öl und einen Fettsäureester umfasst. Dieses Gel enthält dabei mindestens 20 Gew.-% Wasser und mindestens 45 Gew.-% Hydrocolloide.

Darüber hinaus sind hydrophile Salben bekannt, die einen begrenzten Anteil an Wasser aufnehmen und zur Wundversorgung eingesetzt werden können. Diese Salben beinhalten ein Gemisch aus verschiedenen Mono-, Di- und Triglyceriden und einem unpolaren Öl und werden beispielsweise in den Produkten Atrauman® auf Trägermaterialien zur Herstellung von so genannten Salbenkompressen verarbeitet.

Weiterhin ist mit der EP 65 399 eine sterile Wundauflage bekannt, die ein mit einer Wundsalbe imprägniertes Trägermaterial und einen wasserlöslichen Film aus Polyvinylpyrrolidon aufweist. Die Salbe kann eine hydrophile oder hydrophobe Wundsalbe sein.

Mit der WO 96/ 036 315 ist eine sterilisierbare Paste oder Creme bekannt, die eine Emulsion und ein wasserunlösliches, gelbildendes Material umfasst, das vernetzte Carboxymethylcellulose sein kann. Die Emulsion ihrerseits soll ein Öl oder Wax, Wasser und einen Emulgator umfassen, wobei der Wassergehalt mindestens 40 Gew.-% beträgt.

Mit der WO 01/ 070 285 ist eine Kompresse zur Behandlung von Wunden bekannt, die eine hydrophobe Elastomer-Matrix umfasst, in die Hydrocolloidpartikel dispergiert sind. Die Matrix soll weiterhin 55 bis 90 Gew.-% eines unpolaren Öls und ein grenzflächenaktives Mittel mit einem HLB-Wert größer als 10 aufweisen.

Ausgehend vom Stand der Technik ist es eine Aufgabe der vorliegenden Erfindung, eine Wundkontaktschicht bereitzustellen, die in der Lage ist, von einer Wunde abgegebenes Wundexsudat aufzunehmen und den Heilungsprozess einer Wunde optimal zu unterstützen, indem ein Wirkstoff freigesetzt wird. Darüber hinaus soll eine Wundkontaktschicht zur Verfügung gestellt werden, die in der Lage ist, von einer Wunde abgegebenes Wundexsudat aufzunehmen, einen Wirkstoff abzugeben und ein feuchtes Wundheilungsklima zur Verfügung zu stellen. Weiterhin soll eine Wundauflage bereitgestellt werden, die insbesondere bei mäßig bis stark exsudierenden Wunden eingesetzt werden kann.

Gelöst werden diese Aufgaben durch eine Wundkontaktschicht gemäß Anspruch 1.

Hierbei und im Folgenden sind im Zusammenhang mit der vorliegenden Erfindung alle Gehaltsangaben als Gewichts-% in Bezug auf die Gesamtmasse der medizinischen Zusammensetzung zu verstehen, sofern nichts anderes angegeben ist. Des Weiteren soll im Zusammenhang mit der vorliegenden Erfindung als hydrophile Basis eine medizinische Zusammensetzung verstanden sein, die ein- oder mehrphasig ist und durch den Anteil an mindestens einem Emulgator als Emulsion vorliegt oder in der Lage ist eine Emulsion auszubilden. Bei solchen Emulsionen kann es sich um Emulsionen handeln, die mindestens eine Wasser- und/oder Gelphase und mindestens eine Ölphase umfassen.

Ein Vorteil dieser Wundkontaktschicht besteht darin, dass die Zusammensetzung durch den Gehalt an Hydrocolloiden, die in der hydrophilen Basis dispergiert sind, eine besonders große Menge an Flüssigkeiten wie beispielsweise Wundexsudat sehr schnell aufnehmen kann. Die hydrophile Basis bildet dabei bei Kontakt mit Flüssigkeiten innerhalb kurzer Zeit eine Emulsion aus, worauf in einem zweiten Schritt das Wasser in der Emulsion von den in der Basis dispers verteilten Hydrocolloiden aufgenommen wird. Dieser Vorgang kann auch parallel erfolgen. In jedem Fall bilden die Hydrocolloide neben der sich bilden Emulsion einen zweiten Flüssigkeitsspeicher aus. Durch das Trägermaterial wird erreicht, dass die Wundkontaktschicht eine leicht applizierbare Form erhält, die direkt flächig auf eine Wunde aufgebracht werden kann. Dabei kann vorgesehen sein, dass die Zusammensetzung auf mindestens eine Seite des Trägermaterials gestrichen oder in sonstiger Art und Weise aufgebracht ist. Es kann auch vorgesehen sein, dass die Zusammensetzung auf beiden Seiten auf das Trägermaterial aufgebracht ist oder dass das Trägermaterial vollständig mit der Zusammensetzung imprägniert ist. Die Abgabe eines antimikrobiell wirkenden Metalls wird hierbei unter anderem durch die Aufnahme an Wundexsudat gesteuert. Je mehr Wundexsudat von der hydrophilen Basis oder den Hydrocolloiden aufgenommen wird, desto mehr antimikrobielles Metall wird abgegeben. Die Trägerschicht stellt somit ein Depot für das Metall dar, das durch die Zusammensetzung kontrolliert freigesetzt werden kann.

In der bevorzugten Ausführungsform der Wundkontaktschicht soll die hydrophile Basis weniger als 10 Gew.-Wasser umfassen oder weiter bevorzugt wasserfrei sein. Diese hydrophile Basis liegt somit als Einphasengemisch vor und ist durch den Anteil an mindestens einem Emulgator in der Lage, bei Zugabe von beispielsweise Wasser eine Emulsion auszubilden. Hiermit und im Folgenden ist im Zusammenhang mit der vorliegenden Erfindung gemeint, dass die hydrophile Basis Spuren an Wasser enthalten kann, wobei der Gehalt an Wasser dabei höchstens 1 Gew.-% bezogen auf die Masse der hydrophilen Basis betragen soll.

In einer weiteren bevorzugten Ausführungsform weist die Zusammensetzung eine hydrophile Basis auf, die eine Creme, Cremegrundlage oder Salbe ist. Insbesondere ist die hydrophile Basis eine hydrophile Creme oder Cremegrundlage oder eine hydrophile Salbe. Unter einer Salbe soll hierbei und im Kontext dieser Anmeldung ein Einphasensystem verstanden sein, wogegen eine Creme ein Zwei- oder Mehrphasensystem ist. Eine genaue Unterscheidung dieser Formulierungen auch in Abgrenzung von weiteren Formulierungen ist mit dem Deutschen Arzneibuch DAB 9 und dessen Kommentar gegeben auf das/ den hier ausdrücklich Bezug genommen wird.

Es ist vorgesehen, dass die Zusammensetzung eine hydrophile Basis umfasst, die weiterhin 10 bis 30 Gew.-% unpolare Lipide umfasst.

Im Rahmen der vorliegenden Erfindung wird als Oberbegriff für Fette, Öle, Wachse und dergleichen der Ausdruck "Lipide" verwendet. Auch werden die Begriffe "Ölphase" und "Lipidphase" synonym angewendet. Lipide unterscheiden sich unter anderem in ihrer Polarität. Es wurde bereits vorgeschlagen, die Grenzflächenspannung gegenüber Wasser als Maß für die Polarität eines Lipids bzw. einer Lipidphase anzunehmen. Dabei gilt, dass die Polarität der betreffenden Lipidphase umso größer ist, je niedriger die Grenzflächenspannung zwischen dieser Lipidphase und Wasser ist. Erfindungsgemäß wird die Grenzflächenspannung als ein mögliches Maß für die Polarität einer gegebenen Ölkomponente angesehen. Die Grenzflächenspannung ist dabei diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/ Länge und wird gewöhnlich in mN/ m wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden insbesondere Lipide angesehen, deren Grenzflächenspannung gegen Wasser weniger als 20 mN/ m beträgt und als unpolar solche, deren Grenzflächenspannung gegen Wasser insbesondere mehr als 30 mN/ m beträgt. Lipide mit einer Grenzflächenspannung gegen Wasser zwischen 20 und 30 mN/ m werden im Allgemeinen als mittelpolar bezeichnet.

Unpolare Lipide sind insbesondere solche Lipide, welche gewählt werden aus der Gruppe der verzweigten und unverzweigten Kohlenwasserstoffe und Kohlenwasserstoffwachse, insbesondere Vaseline, Petrolatum, Paraffinöl, Mineralöl und Polyisobutene.

Neben unpolaren Lipidkomponenten kann die erfindungsgemäße hydrophile Basis auch polare und mittelpolare Lipide enthalten. Polare oder mittelpolare Lipide sind beispielsweise solche aus der Gruppe der Fettsäuretriglyceride, Fettsäurediglyceride, Fettsäuremonoglyceride oder Fettsäureester Oligomerer des Glycerins wie beispielsweise Voll- oder Partialfettsäureester des Diglycerins oder Triglycerins. Insbesondere können die Tri-, Di- und Monoglyceride Ester gesättigter und/oder ungesättigter, verzweigter und/oder unverzweigter Alkancarbonsäuren einer Kettenlänge von 8 bis 24, insbesondere 12 bis 18 C-Atomen sein. Die Fettsäuretriglyceride, Fettsäurediglyceride oder Fettsäuremonoglyceride können beispielsweise vorteilhaft gewählt werden aus der Gruppe der synthetischen, halbsynthetischen und natürlichen Fette oder Öle.

Als hydrophile Basis wird im Zusammenhang mit der vorliegenden Erfindung insbesondere auch ein Gemisch angesehen, das einen Anteil an polaren und unpolaren Lipiden und zu 0,5 - 30 Gew.-% mindestens einen Emulgator umfasst, wobei der Anteil an mittelpolaren und polaren Lipiden in der hydrophilen Basis im Verhältnis zu unpolaren Lipiden mehr als 1:1 insbesondere mehr als 2:1 und ganz besonders zwischen 3:1 und 10:1 bezogen auf den Gesamtgehalt an Lipiden beträgt.

Die erfindungsgemäße Zusammensetzung weist eine hydrophile Basis auf, die 20 - 80 Gew.-% Mono-, Di- und/ oder Triglyceride und/ oder Voll- oder Partialester Oligomerer des Glycerins bezogen auf das Gesamtgewicht der Zusammensetzung umfasst. Insbesondere umfasst die hydrophile Basis 30 - 70 Gew.-% und ganz besonders 40 - 70 Gew.-% Mono-, Di- und/ oder Triglyceride und/ oder Voll- oder Partialester Oligomerer des Glycerins bezogen auf das Gesamtgewicht der Zusammensetzung auf. Dabei ist es besonders vorteilhaft, wenn die Zusammensetzung 10 - 50 Gew.-% Mono-, Di- und/ oder Triglyceride und 10 - 30 Gew.-% Partialester Oligimerer des Glycerins insbesondere des Diglycerins oder der Triglycerins umfasst.

In einer weiteren besonders bevorzugten Ausführungsform weist eine erfindungsgemäße Zusammensetzung eine hydrophile Basis auf, die 40 - 80 Gew.-% Monoglyceride, Diglyceride, Triglyceride und/ oder Partialester Oligomerer des Glycerins insbesondere des Diglycerins oder des Triglycerins, 15 - 30 Gew.-% unpolare Fette und 0,5 - 30 Gew.-% Emulgator bezogen auf das Gesamtgewicht der Zusammensetzung umfasst.

Als Emulgatoren sollen im Zusammenhang mit der vorliegenden Erfindung solche Substanzen verstanden sein, die eine Grenzflächenaktivenaktivität aufweisen, so dass sich bei Zugabe von Wasser zu der hydrophilen Basis ein Mehrphasengemisch, nämlich eine Emulsion ausbilden kann. Insbesondere soll eine erfindungsgemäße Zusammensetzung mindestens einen Emulgator umfassen, durch den die hydrophile Basis in der Lage ist, bei Zugabe von Wasser eine Wasser-in-Öl-Emulsion (W/O-Emulsion), Gel-in-Öl-Emulsion (G/O-Emulsion), eine Öl-in-Wasser-Emulsion (O/W-Emulsion), Öl-in-Gel-Emulsion (O/G-Emulsion), Wasser-in-Öl-in-Wasser-Emulsion, (W/O/W-Emulsion) Gel-in-Öl-in-Gel-Emulsion (G/O/G-Emulsion), Gel-in-Öl-in Wasser-Emulsion (G/O/W-Emulsion), Wasser-in-Öl-in-Gel-Emulsion (G/O/W-Emulsion), Öl-in-Wasser-in-Öl-Emulsion (O/W/O-Emulsion), oder Öl-in-Gel-in-Öl-Emulsion (O/G/O-Emulsion) auszubilden. Weiterhin bevorzugt sind solche Emulgatoren, die eine O/W- oder W/O-Emulsion oder eine O/G- oder G/O-Emulsion ausbilden können und die frei von Ethylen- oder Propylenglykolen oder Ethylen-PropylenGlykolen sind, das heißt keinerlei Substanzen umfassen, die Ethylen-, Propylen- oder Ethylen-Propylen-Glykol-Einheiten umfassen.

Die erfindungsgemäße Zusammensetzung weist 0,5 - 50 Gew.-% mindestens eines Emulgators, insbesondere 0,5 - 40 Gew.-% mindestens eines Emulgators, insbesondere 0,5 - 30 Gew.-% mindestens eines Emulgators, insbesondere 1 - 20 Gew.-% mindestens eines Emulgators uns ganz besonders bevorzugt 1 - 10 Gew.-% mindestens eines Emulgators auf.

In einer weiteren Ausgestaltung der Erfindung umfasst damit eine erfindungsgemäße Zusammensetzung gemäß Anspruch 1 wobei die hydrophile Basis 0,5 bis 50 Gew.-% mindestens einen O/W-Emulgator umfasst. Es kann jedoch auch vorgesehen sein, dass anstelle des O/W-Emulgator ein nichtionischer W/O-Emulgator eingesetzt wird.

Bei der Verwendung eines Emulgators vom O/W-Typ besteht der Vorteil darin, dass bei der Anwendung der Zusammensetzung die komplette Zusammensetzung besonders leicht mittels Wasser beispielsweise von einer Wunde abgewaschen werden kann.

Weiterhin bevorzugt kann eine erfindungsgemäße Zusammensetzung mindestens einen nichtionischen Emulgator mit einem HLB-Wert von 3 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionische O/W- Emulgatoren mit einem HLB-Wert von 10 bis 15 sowie nichtionische W/O-Emulgatoren mit einem HLB-Wert von 3 bis 6 sind erfindungsgemäß besonders bevorzugt.

Vorteilhaft kann oder können der oder die Emulgatoren insbesondere nichtionische O/W-Emulgatoren gewählt werden aus der Gruppe:
- der Fettalkoholethoxylate mit der allgemeinen Formel R-O-(CH₂-CH₂-O)ₙ-H oder der Fettalkoholpropoxylate mit der allgemeinen Formel R-O-(CH₂-CH(CH₃)-O)ₙ-H, wobei R einen verzweigten oder unverzweigten Alkyl- oder Alkenylrest und n eine Zahl von 10 bis 50 darstellen,
- der ethoxylierten oder propoxylierte Wollwachsalkohole,
- der Polyethylenglykolether mit der allgemeinen Formel R-O-(CH₂-CH₂-O)ₙ-R' oder der Polypropylenglykolether mit der allgemeinen Formel R-O-(CH₂-CH(CH₃)-O)ₙ-R', wobei R und R' unabhängig voneinander verzweigte oder unverzweigte Alkyl- oder Alkenylreste und n eine Zahl von 10 bis 80 darstellen,
- der Fettsäureethoxylate mit der allgemeinen Formel R-COO-(CH₂-CH₂-O)"H oder der Fettsäurepropoxylate mit der allgemeinen Formel R-COO-(CH₂-CH(CH₃)-Oₙ)-H, wobei R einen verzweigten oder unverzweigten Alkyl- oder Alkenylrest und n eine Zahl von 10 bis 40 darstellen,
- der veretherten Fettsäureethoxylate mit der allgemeinen Formel R-COO-(CH₂-CH₂-O)ₙ-R' oder der veresterten Fettsäurepropoxylate mit der allgemeinen Formel R-COO-(CH₂-CH(CH₃)-O)ₙ-R', wobei R und R' unabhängig voneinander verzweigte oder unverzweigte Alkyl- oder Alkenylreste und n eine Zahl von 10 bis 80 darstellen,
- der veresterten Fettsäureethoxylate mit der allgemeinen Formel R-COO-(CH₂-CH₂-O)ₙ-C(O)-R' oder der veresterten Fettsäurepropoxylate mit der allgemeinen Formel R-COO-(CH₂-CH(CH₃)-O)ₙ-C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester oder der Polypropylenglycolglycerinfettsäureester gesättigter und/oder ungesättigter, verzweigter und/oder unverzweigter Fettsäuren und einem Ethoxylierungsgrad oder Propoxylierungsgrad zwischen 3 und 50,
- der ethoxylierten oder propoxylierter Sorbitanester mit einem Ethoxylierungsgrad oder Propoxylierungsgrad von 3 bis 100,
- der ethoxylierten oder propoxylierte Triglyceride mit einem Ethoxylierungsgrad oder Propoxylierungsgrad zwischen 3 und 150,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth- Typ.

Vorteilhaft im Rahmen der vorliegenden Erfindung einsetzbare Emulgatoren sind weiterhin nichtionische W/O-Emulgatoren aus der Gruppe der Dicarbonsäureester oder Tricarbonsäureester. Insbesondere sind hiervon Ester der Malonsäure, Bernsteinsäure, Adipinsäure geeignet. Weiterhin bevorzugt sind hiervon Ester der Dicarbonsäuren, insbesondere Ester der Bernsteinsäure geeignet, die mit gesättigten oder ungesättigten und/ oder linearen oder verzweigten C8-C24-Fettalkohole und/ oder Glycerin sowie deren Oligomeren, insbesondere Diglycerin oder Triglycerin gebildet werden. Insbesondere haben sich als nichtionische W/O-Emulgatoren Ester der Bernsteinsäure mit gesättigten und verzweigten C8-C24-Fettalkohole und/ oder Glycerin sowie deren Oligomeren, insbesondere Diglycerin oder Triglycerin als besonders vorteilhaft erwiesen. Ganz besonderes sind hiervon Dicarbonsäureester geeignet, die aus Bernsteinsäure und gesättigten und verzweigten C8-C24-Fettalkoholen und Diglycerin gebildet werden. Ein solcher Emulgator wird gemäß der INCI-Nomenklatur als Isostearyl Digyceryl Succinate bezeichnet und ist unter der Produktbezeichnung "Imwitor®780" erhältlich. Diese Emulgatoren haben den weiteren Vorteil, dass sie polyethylenglykol-frei sind, das heißt keine Einheiten von Ethylenglykol umfassen.

Als O/W-Emulgatoren können im Zusammenhang mit der vorliegenden Erfindung insbesondere auch ionische O/W-Emulgatoren verwendet werden. Als O/W-Emulgatoren insbesondere ionische O/W-Emulgatoren können vorteilhafterweise Emulgatoren ausgewählt werden aus der Gruppe der Ester von Monoglyceriden und/oder Diglyceriden gesättigter oder ungesättigter Fettsäuren mit Hydroxycarbonsäuren und/oder Tricarbonsäuren. Besonders bevorzugt sind als O/W-Emulgatoren partiell neutralisierte Ester von Monoglyceriden und/oder Diglyceriden gesättigter Fettsäuren mit Hydroxycarbonsäuren und/ oder Tricarbonsäuren, insbesondere der Milchsäure und/ oder der Zitronensäure. Ganz besonders bevorzugt sind Ester der Milchsäure und/ oder Zitronensäure die gemäß der INCI-Nomenklatur als Glyceryl Cocoate Citrate Lactate bezeichnet werden. Solche Emulgatoren sind beispielsweise unter der Produktbezeichnung "IMWITOR® 380" oder "IMWITOR® 377" erhältlich. Diese Emulgatoren haben den weiteren Vorteil, dass sie polyethylenglykol-frei sind, das heißt keine Einheiten von Ethylenglykol umfassen.

Gemäß der vorliegenden Erfindung soll unter einem Hydrocolloid ein Material verstanden sein, das ein hydrophiles synthetisches oder natürliches Polymermaterial ist, das löslich oder absorbierend und/ oder quellend in Wasser ist und ein Gel bildet. Vorzugsweise umfasst eine erfindungsgemäße Zusammensetzung ein Hydrocolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum oder Gelatine. Ganz besonders bevorzugt können als Hydrocolloid Cellulose oder Derivate oder deren Salze, Alginsäure oder deren Derivate oder Salze sowie Gemische hiervon verwendet werden.

Das Hydrocolloid kann sowohl in Form von Fasern als auch in Form von Partikeln und/ oder Fasern dispergiert in der Zusammensetzung vorliegen. Insbesondere kann das Hydrocolloid in Form von Partikeln vorliegen. Der Anteil an Hydrocolloiden in der Zusammensetzung beträgt 5 bis 40 Gew.-% bezogen auf das Gesamtgewicht der Zusammensetzung. Vorzugsweise kann der Anteil an Hydrocolloiden 5 bis 30 Gew.-%, weiterhin vorzugsweise 10 bis 25 Gew.-% und ganz besonders bevorzugt 15 bis 25 Gew.-% bezogen auf das Gesamtgewicht der Zusammensetzung betragen.

Besonders bevorzugt sind Hydrocolloide, die in Partikelform vorliegen, wobei die Partikel einen Wassergehalt von weniger als 10 Gew.-% bezogen auf die Hydrocolloidpartikel aufweisen.

Weiterhin bevorzugt können Hydrocolloide verwendet werden, die inter- und/ oder intramolekular vernetzt oder quervernetzt sind. Diese Hydrocolloide sind nicht löslich in beispielsweise Wasser oder Salzlösungen, das heißt diese Hydrocolloide quellen bei Zugabe dieser Flüssigkeiten und weisen einen inneren Zusammenhalt auf, dass die gequollenen Partikel dispers in der Zusammensetzung vorliegen.

Gemäß einer besonders bevorzugten Ausführungsform umfasst eine erfindungsgemäße Zusammensetzung vorzugsweise mindestens ein Hydrocolloid ausgewählt aus der Gruppe der Cellulose-Derivate oder deren Salze, Alginate oder deren Derivate, Chitin oder dessen Derivate oder dessen Salze. Hierbei ist der Ursprung der Hydrocolloide unbeachtlich, das heißt, dass diese Hydrocolloide pflanzlichen oder tierischen Ursprungs sein können oder auf synthetischem Weg beispielsweise durch mikrobiologische Verfahren hergestellt sein können. Es ist auch möglich, Hydrocolloide zu verwenden, die pflanzlichen oder tierischen Ursprungs und durch chemische Synthese modifiziert sind.

Zu der Gruppe der Cellulose-Derivate zählen im Zusammenhang mit der vorliegenden Erfindung insbesondere Celluloseether und Celluloseester sowie deren Salze. Als Celluloseether kommen hierbei insbesondere Hydroxyalkylcellulosen insbesondere Hydroxy-C₁₋₆-alkylcellulose wie zum Beispiel Hydroxymethylcellulose, Hydroxyethylcellulose, Hydroxypropylcellulose oder Hydroxybutylcellulose und ganz besonders bevorzugt Hydroxymethylcellulose oder Hydroxyethylcellulose zum Einsatz. Als Celluloseester kommen insbesondere Carboxyalkylcellulose insbesondere Carboxy-C₁₋₆-alkylcellulose wie zum Beispiel Carboxymethylcellulose, Carboxyethylcellulose, Carboxypropylcellulose oder Carboxybutylcellulose odere deren Salze und ganz besonders bevorzugt Carboxymethylcellulose oder Carboxyethylcellulose oder deren Salze zum Einsatz.

Gemäß einer weiteren bevorzugten Ausführungsform umfasst die Zusammensetzung mindestens zwei verschiedene Hydrocolloide. Dabei hat es sich als besonders vorteilhaft erwiesen, wenn die mindestens zwei Hydrocolloide ausgewählt werden aus der Gruppe der Cellulose-Derivate oder deren Salze insbesondere Celluloseester oder deren Salze, Alginate oder deren Derivate, Chitin oder dessen Derivate oder dessen Salze. Weiterhin ist auch eine Wundsalbe umfassend eine medizinische Zusammensetzung gemäß Anspruch 1, Gegenstand der vorliegenden Erfindung. Diese Wundsalbe kann besonders zur Anwendung bei mäßig bis stark nässenden Wunden zur Anwendung gebracht werden, da mit dieser Wundsalbe einerseits in besonderem Maß Wundexsudat aufgesaugt wird und andererseits durch den Anteil an Hydrocolloiden, die einen Speicher für Flüssigkeiten bilden, ein feuchtes Klima einer Wunde bereitgestellt wird. Die Hydrocolloide wirken in der Wundsalbe zugleich als Flüssigkeitsspeicher als auch als Feuchtigkeitsspender.

Gemäß einem weiterführenden Gedanken der vorliegenden Erfindung ist auch eine Wundkontaktschicht umfassend ein Trägermaterial und eine medizinische Zusammensetzung Gegenstand der vorliegenden Erfindung. Insbesondere ist eine Wundkontaktschicht umfassend ein Trägermaterial und eine Zusammensetzung gemäß Anspruch 1 Gegenstand der vorliegenden Erfindung. Durch das Trägermaterial wird erreicht, dass die Wundkontaktschicht eine leicht applizierbare Form erhält, die direkt flächig auf eine Wunde aufgebracht werden kann. Dabei kann vorgesehen sein, dass die Zusammensetzung auf mindestens eine Seite des Trägermaterials gestrichen oder in sonstiger Art und Weise aufgebracht ist. Es kann auch vorgesehen sein, dass die Zusammensetzung auf beiden Seiten auf das Trägermaterial aufgebracht ist oder dass das Trägermaterial vollständig mit der Zusammensetzung imprägniert ist.

Ein weiterer Vorteil gegenüber bekannten Wundkontaktschichten besteht darin, dass bei der Applikation auf eine Wunde, die normalerweise von geschultem Personal mittels Handschuhen durchgeführt wird, eine erfindungsgemäße Wundkontaktschicht nicht an den Handschuhen haftet oder klebt. Somit sind diese Wundkontaktschichten besonders handhabungssicher.

Durch den Anteil an Fettsäureglyceriden in der Zusammensetzung wird der eine Wunde umgebenden Haut, der sogenannten peripheren Wundhaut, pflegende Bestandteile bereitgestellt, die eine Wundheilung in besonderem Maße unterstützen.

Als Trägermaterial können hierbei verschiedenste Materialien eingesetzt werden. Insbesondere hat sich herausgestellt, dass hierzu Polymerfilme oder -folien, Polymerschäume, Nonwoven sowie textile Materialien eingesetzt werden können. Als Trägermaterial in einer erfindungsgemäßen Wundkontaktschicht können insbesondere Nonwoven sowie textile Materialien wie Gestricke, Gewirke oder Gewebe eingesetzt werden. Ganz besonders bevorzugt können hierbei hydrophobe Gestricke, Gewirke oder Gewebe, die selbst keine Flüssigkeiten absorbieren verwendet werden. Insbesondere umfasst eine erfindungsgemäße Wundkontaktschicht als Trägermaterial ein Polyamid-Gewirk.

Wird ein textiles Trägermaterial eingesetzt, so kann dass Material insbesondere auch mit Öffnungen versehen sein, das heißt, dass das Trägermaterial mit Löchern versehen ist oder in Gitterform vorliegt. Insbesondere ist vorgesehen, dass das Trägermaterial ein Gewirk, Gewebe oder Gestrick ist, das Löcher aufweist, deren maximale lichte Weite im Bereich von 0,3 bis 3,0 mm vorzugsweise von 0,5 bis 2,5 mm und besonders bevorzugt von 0,5 bis 2,0 mm im ungedehnten Zustand des Materials beträgt. Hierbei können die Löcher jede beliebige Form annehmen wie z.B. kreisförmig, elliptisch, quadratisch, sechseckig oder achteckig. Diese Gewirke weisen ein Flächengewicht von mindestens 20 g/ m² bis höchstens 120 g/ m² auf.

Weiterhin kann vorgesehen sein, durch die Wundkontaktschicht mindestens eine die Wundheilung fördernde Substanz freizusetzen. Hierzu zählen insbesondere Substanzen, die fungizid, bakterizid oder antimikrobiell wirken. In einer besonderen Ausführungsform umfasst die Wundkontaktschicht ein Hydrocolloid, das seinerseits mindestens ein fungizid, bakterizid oder antimikrobiell Substanz umfasst. Ganz besonders sind hierbei Chitosan, Silber, Silberkomplexe, Silbersalze, Zink, Zinksalze oder Zinkkomplexe geeignet.

Es kann jedoch auch vorgesehen sein, dass ein wundheilungsförderndes Mittel direkt auf das Trägermaterial aufgebracht ist. In besonders vorteilhafter Weise wird in einer weiteren Ausführung der Wundkontaktschicht als Trägermaterial ein Nonwoven oder ein textiles Material wie Gestrick, Gewirk oder Gewebe verwendet, das mit einem antimikrobiell wirkenden Metall, vorzugsweise Silber oder Silbersalzen, beschichtet ist. Bei der Verwendung eines solchen Trägermaterials kann die Zusammensetzung direkt auf eine erste Seite des Trägermaterials auf das Metall oder Metallsalz aufgebracht werden. Dabei ist es besonders vorteilhaft, wenn die Zusammensetzung wasserfrei ist.

Darüber hinaus kann auch vorgesehen sein, dass das antimikrobiell wirkende Metall als Beschichtung auf das Trägermaterial aufgebracht oder auf bzw. in den Träger imprägniert oder inkorporiert ist. Dabei kann insbesondere vorgesehen sein, dass als antimikrobielles Metall elementares Silber, Silbersalze oder Silberkomplexe verwendet werden.

Weiterhin kann vorgesehen sein, dass die Wundkontaktschicht ein Nonwoven als Trägermaterial umfasst, das aus einem Fasergemisch aus antimikrobiell Metall umfassenden Fasern und metallfreien Fasern besteht.

Nach einer besonders bevorzugten Ausführungsform, kann vorgesehen sein, dass das Trägermaterial mehrschichtig ist. In diesem Fall kann vorgesehen sein, dass lediglich eine der Schichten des Trägermaterials ein antimikrobielles Metall aufweist, das durch die Wundkontaktschicht freisetzbar ist. Alternativ können antimikrobielle Metalle gleicher oder verschiedener Art in mehreren Schichten untergebracht sein.

Insbesondere kann vorgesehen sein, dass die Wundkontaktschicht weniger als 500 µg/ 100cm² in 24h bei 37°C in 100 ml Wasser an antimikrobiellem Metall freisetzt, insbesondere weniger als 400 µg/ 100cm² in 24h bei 37°C in 100 ml Wasser und insbesondere weniger als 300 µg/100cm² in 24h bei 37°C in 100 ml Wasser freisetzt. Jedoch kann insbesondere vorgesehen sein, das die Wundauflage mehr als 2 µg/ 100cm² in 24h bei 37°C in 100 ml Wasser freisetzt. Zur Bestimmung der abgegebenen Menge Silber wird die Atomabsorptionsspektroskopie herangezogen.

Dass das Trägermaterial mindestens auf seiner ersten Seite eine Zusammensetzung umfasst, ist dabei erfindungsgemäß so zu verstehen, dass die Zusammensetzung entweder unmittelbar auf dem mit Metall versehenen Träger angeordnet ist oder zunächst auf der ersten Seite eine kontinuierliche oder diskontinuierliche Metallschicht angebracht ist, auf die dann wiederum die Zusammensetzung aufgebracht ist. Es kann auch ein beidseitiges Aufbringen der Zusammensetzung erwünscht sein, insbesondere wenn ein Eintamponieren der Wundauflage vorgenommen werden soll. In diesem Fall ist es vorteilhaft, wenn auch der Metallauftrag beidseitig oder das Gewirk umschließend aufgebracht ist.

Dabei wirkt die Zusammensetzung, die insbesondere eine Salbe oder Creme sein kann, als Vermittler zwischen dem mit dem Metall ausgerüsteten Trägermaterial und der Wunde des Patienten. Auf diese Weise kann ein unmittelbarer Kontakt der Wunde mit dem Metall und insbesondere auch ein Verkleben desselben sicher vermieden werden. Darüber hinaus kann diese Salbe oder Creme im Bereich der peripheren Wundhaut eine pflegende Wirkung erzeugen. Tritt die Wundkontaktschicht dann über die Zusammensetzung mit der Wunde in Kontakt, wird insbesondere über die Wundflüssigkeit durch Vermittlung der Zusammensetzung das Metall, z. B. Silber aus der Wundauflage freigesetzt und gelangt über die Zusammensetzung in die Wunde. Dabei kann insbesondere vorgesehen sein, dass als Metall elementares Silber eingesetzt wird. Das Metall kann als Beschichtung auf dem Trägermaterial angeordnet sein oder in den Träger einimprägniert werden.

Besonders vorteilhaft ist ein Auftrag einer Zusammensetzung, insbesondere einer Salbe, Creme oder Cremegrundlage, auf einem Trägermaterial in einer Menge von mindestens 50 g/ m², insbesondere von mindestens 100 g/ m² und besonders bevorzugt von 100 bis 450 g/ m² und ganz besonders bevorzugt von 100 bis 300 g/ m² vorzusehen.

Weiterhin umfasst die vorliegende Erfindung eine Wundauflage, die eine Abdeckschicht und eine Wundkontaktschicht umfasst. Die Wundkontaktschicht umfasst dabei eine Zusammensetzung oder eine Wundsalbe gemäß Anspruch 1.

Gemäß einer Weiterbildung der Erfindung umfasst die vorliegende Erfindung auch eine Wundauflage, die eine Abdeckschicht, eine absorbierende Schicht und eine Wundkontaktschicht umfasst, wobei die Wundkontaktschicht eine Zusammensetzung gemäß Anspruch 1 umfasst.

Als Abdeckschicht kann eine Wundauflage insbesondere eine Polymerfolie oder einen Polymerfilm aufweisen. Ganz besonders bevorzugt sind Polymerfilme, die eine hohe Wasserdampfdurchlässigkeit aufweisen. Hierzu sind besonders Polyurethan-, Polyetherurethan-, Polyesterurethan-, Polyether-Polyamid-Copolymer-, Polyacrylat- oder Polymethacrylat-Filme geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms der Wundauflage weist vorzugsweise mindestens 750 g/ m²/ 24 Std., insbesondere mindestens 1000 g/ m²/ 24 Std. und ganz besonders bevorzugt mindestens 2000 g/ m²/ 24 Std. auf (gemessen nach DIN 13726).

Darüber hinaus kann vorgesehen sein, dass eine erfindungsgemäße Wundauflage als so genanntes Inseldressing vorliegt. Hierbei weist die Wundkontaktschicht eine kleinere Auflagefläche auf als die Abdeckschicht, d.h. die Wundkontaktschicht ist entlang ihres Umfangs von einer Abdeckschicht umgeben. Dabei kann die Abdeckschicht ein Haftklebemittel aufweisen oder klebend ausgerüstet sein, so dass die gesamte Wundauflage auf der Haut eines Patienten haften oder kleben kann. Dieser Auftrag des Haftklebemittels kann sowohl vollflächig als auch diskontinuierlich oder nur in bestimmten Bereichen erfolgen. Bei dem verwendeten Klebemittel kann es sich um einen üblichen Haftkleber, insbesondere um einen Acrylathaftkleber oder einen druckempfindlichen Haftkleber auf Basis von Polyurethanen handeln. Bevorzugt handelt es sich um Gelhaftkleber, insbesondere auf Basis von Polyurethanen, insbesondere wässrigen Polyurethanen. Ganz besonders bevorzugt handelt es sich um Hydrogelhaftkleber, insbesondere auf Basis von wässrigen Acrylaten.

Gemäß einer Weiterführung der Erfindung kann die Wundauflage eine Abdeckschicht aufweisen, die vollflächig mit einem Haftklebemittel beschichtet ist. Die Wasserdampfdurchlässigkeit dieser mit dem Haftklebemittel versehenen Trägerschicht beträgt dabei vorzugsweise mindestens 1000 g/ m²/ 24 Std., besonders bevorzugt mindestens 1200 g/ m²/ 24 Std. und ganz besonders bevorzugt mindestens 2000 g/ m²/ 24 Std. (gemessen nach DIN EN 13726).

Eine erfindungsgemäße Wundauflage kann in jeder geometrischen Form beispielsweise in dreieckiger, runder, ovaler oder quadratischer, rechteckiger oder jeder symmetrischen oder unsymmetrischen Form vorliegen.

Es kann weiterhin vorgesehen sein, das eine erfindungsgemäße Wundauflage weitere Schichten aufweist, die unterschiedliche Funktionen besitzen können. Gemäß einer Weiterentwicklung der vorliegenden Erfindung weist die Wundauflage mindestens eine weitere Schicht auf. Diese Schicht kann bevorzugt eine Releaseschicht zum Schutz vor Verschmutzungen sein, die auf der im anwendungsgerechten Zustand der Wundauflage auf der Wunde zu liegende Seite der Wundkontaktschicht aufgebracht ist. Es kann auch vorgesehen sein, dass die Wundauflage mindestens eine weitere Schicht zwischen der Wundkontaktschicht und der Abdeckschicht aufweist. Diese weitere Schicht kann eine absorbierende Schicht wie beispielsweise eine absorbierende Schicht aus einem hydrophilen Schaummaterial aus beispielsweise Polyurethan sein.

In einer besonderen Ausgestaltung der Erfindung ist vorgesehen, dass eine erfindungsgemäße Wundauflage in einer Verpackung angeordnet ist. Insbesondere ist dabei vorgesehen, dass die Verpackung eine sterile Verpackung ist. In einer weiteren besonderen Ausgestaltung der Erfindung ist vorgesehen, dass ein System umfassend eine Wundkontaktschicht der beschriebenen Art und eine separate Wundauflage in einer Verpackung angeordnet sind. Insbesondere ist dabei auch vorgesehen, dass die Verpackung eine sterile Verpackung ist. In einer besonders bevorzugten Ausführung dieses Systems liegt in der Verpackung oder in der sterilen Verpackung jeder einzelne Bestandteil oder jede Gruppe von Bestandteilen jeweils separat in Unterverpackungen vor. Es kann auch vorgesehen sein, dass jede Unterverpackung eine sterile Unterverpackung ist.

Nachstehend wird die Erfindung unter Bezugnahme auf die Zeichnungen und die Beispiele erläutert. In den Zeichnungen zeigt:
Figur 1: Wundkontaktschicht im Querschnitt
Figur 2: Wundauflage im Querschnitt
Figur 3: alternative Wundauflage im Querschnitt

### Beispiele

### 1) Zusammensetzung 1

| Nr. | Handelsname | Bezeichnung - INCI, Funktion | Gehalt Gew.-% |
|---|---|---|---|
| 1 | IMWITOR 780 K (Fa. Sasol, Witten - Deutschland) | Isostearyl Diglyceryl Succinate, nichtionischer W/O-Emulgator HLB 3,7 | 5,0 |
| 2 | IMWITOR 900 K (Fa. Sasol, Witten - Deutschland) | Glyceryl Stearate, Co-Emulgator | 4,0 |
| 3 | SOFTISAN 100 (Fa. Sasol, Witten - Deutschland) | Hydrogenated Coco-Glycerides, polares Fett | 4,0 |
| 4 | SOFTISAN 378 (Fa. Sasol, Witten - Deutschland) | Caprylic/Capric/Myristic/Stearic Triglyceride, polares Fett | 23,0 |
| 5 | SOFTISAN 649 (Fa. Sasol, Witten - Deutschland) | Bis-Diglyceryl Polyacyladipate-2, polares Fett | 19,0 |
| 6 | MERKUR Vaseline 115 (Fa. Merkur Vaseline GmbH & Co. KG, Hamburg - Deutschland) | Petrolatum, unpolares Lipid | 25,0 |
| 7 | Blanose 7H3SXF (Fa. Herkules - Deutschland) | Cellulose Gum, Natriumcarboxymethylcellulose, Hydrocolloid | 20,0 |

### Herstellung der Zusammensetzung 1:

Phase A (Komponenten 1 bis 6) wird bei ca. 75-80°C geschmolzen und verrührt. Anschließend wird Phase B (Komponente 7) unter starkem Rühren in Phase A dispergiert. Die Salbenmasse wird unter starkem Rühren abgekühlt, so dass eine feine Kristallstruktur entsteht. Der Tropfpunkt der Zusammensetzung beträgt 46 °C (bestimmt nach Ph. Eur. 2002, Methode 2.2.17).

### 2) Zusammensetzung 2

| Nr. | Handelsname (Hersteller) | Bezeichnung - INCI, Funktion | Gehalt Gew.-% |
|---|---|---|---|
| 1 | IMWITOR 377 (Fa. Sasol, Witten - Deutschland) | Glyceryl Laurate Citrate, ionischer O/W-Emulgator | 5,0 |
| 2 | IMWITOR 900 K (Fa. Sasol, Witten - Deutschland) | Glyceryl Stearate, Co-Emulgator | 4,0 |
| 3 | SOFTISAN 100 (Fa. Sasol, Witten - Deutschland) | Hydrogenated Coco-Glycerides, polares Fett | 4,0 |
| 4 | SOFTISAN 378 (Fa. Sasol, Witten - Deutschland) | Caprylic/Capric/Myristic/Stearic Triglyceride, polares Fett | 23,0 |
| 5 | SOFTISAN 649 (Fa. Sasol, Witten - Deutschland) | Bis-Diglyceryl Polyacyladipate-2, polares Fett | 19,0 |
| 6 | MERKUR Vaseline 115 (Fa. Merkur Vaseline GmbH & Co. KG, Hamburg - Deutschland) | Petrolatum, unpolares Lipid | 25,0 |
| 7 | Blanose 7H3SXF (Fa. Herkules - Deutschland) | Cellulose Gum, Natriumcarboxymethylcellulose, Hydrocolloid | 20,0 |

### Herstellung der Zusammensetzung 2:

Phase A (Komponenten 1 bis 6) wird bei ca. 75-80°C geschmolzen und verrührt. Anschließend wird Phase B (Komponente 7) unter starkem Rühren in Phase A dispergiert. Die Salbenmasse wird unter starkem Rühren abgekühlt, so dass eine feine Kristallstruktur entsteht. Der Tropfpunkt der Zusammensetzung beträgt 48 °C (bestimmt nach Ph. Eur. 2002, Methode 2.2.17).

### 3) Wundkontaktschicht 1

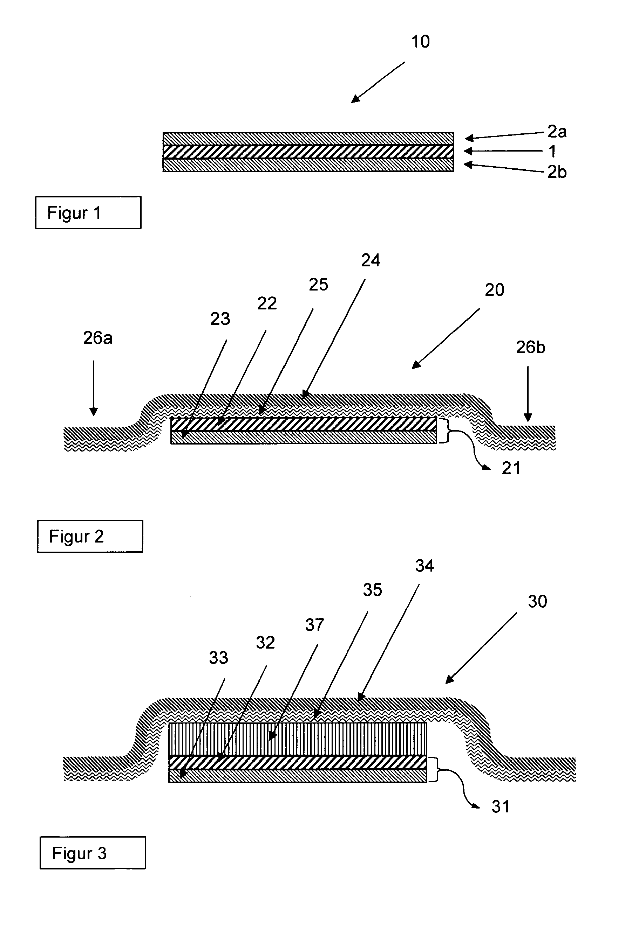

Die vorliegende Wundkontaktschicht hat den mit Figur 1 wiedergegebenen Aufbau. Demnach weist die Wundkontaktschicht (10) ein Trägermaterial (1) auf, das auf beiden Seiten bzw. Oberflächen mit einer Zusammensetzung (2a und 2b) laut Beispiel 1 bestrichen ist. Das Trägermaterial (1) besteht aus einem mit elementarem Silber beschichteten Polyamidträgergewirk, das aus Nylon 6 Trilobalfäden mit 50 µm Kantenlänge hergestellt ist. Das Rohmaterial (ohne Versilberung) hat ein Flächengewicht von 25 bis 30 g/m² und eine Fadenfeinheit von dtex= 22/1. In dem Material sind auf einem Quadratmeter zwischen 11.360 und 13.640 m Faden verwirkt. Dies bedeutet, dass die Oberfläche zwischen 1,7 m² und 2,04 m² liegt. Die Schichtdicke des Silbers auf dem Trägermaterial beträgt ca. 700 nm. Zwischen versilbertem und unversilbertem Produkt ist mit den herkömmlichen Mitteln (Lichtmikroskop) kein Unterschied in der Dreiecksseitenlänge feststellbar. Das Flächengewicht des versilberten Gewirks beläuft sich auf 35 +/- 3 g/m². Dabei beträgt der Gehalt an elementarem Silber ca. 130 mg/ g Polyamidträgergewirk. Derartige silberbeschichtete Gewirke werden von der Firma Statex, Bremen unter dem Namen Shieldex® Tüll Typ Charmeuse vertrieben und wurden hier als Trägermaterial eingesetzt. Die Zusammensetzung benetzt vollständig das Trägermaterial, wobei die Auftragsmenge 280 g/ m² beträgt. Die Wundkontaktschicht weist einen guten Zusammenhalt auf und lässt sich besonders gut auf einer zu behandelnden Wunde applizieren.

### 4) Wundkontaktschicht 2 (nicht erfindungsgemäß)

Auch diese Wundkontaktschicht weist einen mit Figur 1 gezeigten Aufbau auf. Bei dieser Wundkontaktschicht (10) weist die Zusammensetzung die mit Beispiel 1 angegebenen Bestandteile auf. Das Trägermaterial (1) besteht aus einem hydrophoben 100%-igen Polyamid-Gewirk (Fa. Theodor Preuss GmbH & Co. KG, Ubstadt-Weiher- Deutschland) mit einem Flächengewicht von ca. 90 g/ m² (ungedehnt) und weist ca. 46 sechseckigen Öffnungen pro 100 cm auf (in Figur 1 nicht dargestellt). Die maximale lichte Weite der Öffnungen beträgt 0,8 - 1,0 mm. Das Auftragsgewicht der Zusammensetzung beträgt 240 g/ m². Die Beschichtung des textilen Trägermaterials mit der hydrophilen Zusammensetzung erfolgt, indem das Trägermaterial über eine Umlenkwalze durch ein warmes Vorratsbad (40 °C) der hydrophilen Zusammensetzung 1 geführt wird. Nach dem Durchlaufen des Bades wird die überschüssige Menge an übertragener Zusammensetzung mit Hilfe einer Quetschwalze abgestreift. Das beschichtete Material wird auf Raumtemperatur gebracht, konfektioniert, verpackt und sterilisiert.

### 5) Wundkontaktschicht 3 (nicht erfindungsgemäß)

Auch diese Wundkontaktschicht weist einen Aufbau wie mit Figur 1 auf. Bei dieser Wundkontaktschicht (10) weist die Zusammensetzung die mit Beispiel 2 angegebenen Bestandteile auf. Das Trägermaterial (1) besteht aus einem hydrophoben 100%-igen Polyamid-Gewirk (Fa. Theodor Preuss GmbH & Co. KG, Ubstadt-Weiher- Deutschland) mit einem Flächengewicht von ca. 80 g/ m² (ungedehnt) und weist ca. 40 sechseckigen Öffnungen pro 100 cm auf (in Figur 1 nicht dargestellt). Die maximale lichte Weite der Öffnungen beträgt 1,2-1,5 mm. Das Auftragsgewicht der Zusammensetzung beträgt ca. 330 g/ m².

Die Beschichtung des textilen Trägermaterials mit der hydrophilen Zusammensetzung erfolgt, indem das Trägermaterial über eine Umlenkwalze durch ein warmes Vorratsbad (60 °C) der hydrophilen Zusammensetzung 2 geführt wird. Nach dem Durchlaufen des Bades wird die überschüssige Menge an übertragener Zusammensetzung mit Hilfe einer Quetschwalze abgestreift. Das beschichtete Material wird auf Raumtemperatur gebracht, konfektioniert, verpackt und sterilisiert.

Messung der Wasseraufnahme von Wundkontaktschichten im Vergleich zu handelsüblichen Produkten an einem simulierten Wundmilieu:

Der Hintergrund dieses Tests besteht darin, Hinweise darüber zu bekommen, wie sich eine Wundkontaktschicht auf einer Wunde zum Beispiel einer mäßig stark exsudierenden oder stark exsudierenden Wunde verhält.
a) Die Gelatine-Lösung wird wie folgt hergestellt:
   i) Herstellung einer Lösung A:
      In einem ein Liter Messzylinder werden bei Raumtemperatur 0,277 g Kalziumchlorid und 8,298 g Natriumchlorid vorgelegt und mit deionisiertes Wasser auf 1 Liter aufgefüllt. Die Lösung wird solange gerührt bis die Salze gelöst sind.
   ii) Herstellung der Gelatine-Lösungen
      Bei Raumtemperatur werden zu y g der oben genannten Lösung A zur Herstellung einer x %-igen Gelatine Lösung x g Gelatine-Pulver (Type A aus der Schweinehaut, 175 Bloom, GELITA Gelatine, DGF Stoess AG, 69402 Eberbach) gegeben. Hierzu wird die Gelatine auf einmal und schnell in die Lösung A gegeben, die Lösung kräftig geschüttelt, so dass alle Partikel mit der Lösung benetzt sind und das resultierende Gemisch 24 Std. auf dem Wasserbad bei 60 °C gerührt. Es ist sicherzustellen, dass kein Wasser entweicht. Auf diese Weise werden 20 %-ige (x=20, y=80) und 35 %-ige (x=35, y=75) Gelatine-Lösungen hergestellt.
b) Nach 24 Std. werden Petrischalen mit einem Durchmesser von 9 cm mit 30 g der noch warmen Gelatine-Lösung befüllt, mit dem zugehörigen Deckel verschlossen und auf Raumtemperatur abgekühlt. Die resultierenden festen Gele werden zur Untersuchung der Teststücke verwendet.
c) Zur Untersuchung der Wundkontaktschichten werden 3 x 3 cm große Stücke, untersucht, indem diese Teststücke mit einer Seite vollflächig in die mit der Gelatine-Lösung befüllten Petrischalen gelegt werden, die Petrischale mit dem zugehörigen Deckel verschlossen und bei Raumtemperatur 24 Std. belassen werden. Nach 24 Std. wird die aufgenommene Menge Flüssigkeit durch Wiegen der Teststücke bestimmt wird. Hierbei ist darauf zu achten, dass die Masse der Teststücke als ganzes bestimmt wird. Notfalls muss ein auf der Gelatineoberfläche verbleibender Rest mit einem geeigneten Spachtel vorsichtig entfernt werden und bei der Wägung berücksichtigt werden. Die Ergebnisse sind in Tabelle 1 und Tabelle 2 wiedergegeben, wobei zu jeder Probe drei Messungen durchgeführt wurden und jedes Teststück separat in einer Petrischale lag.

### Zur Untersuchung standen:

Probe 1: eine Wundkontaktschicht laut Beispiel 4, mittels Beta-Strahlung sterilisiert (40 kGy)
Probe 2: eine Wundkontaktschicht laut Beispiel 5, mittels Beta-Strahlung sterilisiert (40 kGy)
Probe 3: Urgotül®, Fa. Urgo
Probe 4: Physiotulle®, Fa. Coloplast

**Tabelle 1: Testung auf 20 %-ige Gelatine-Lösung**

| Probe | Einwaage der Probe | Auswaage der Probe nach 24 Std. | Wasseraufnahme nach 24 Std. | Wasseraufnahme nach 24 Std. (bezogen auf Einwaage) | |
|---|---|---|---|---|---|
| | /g | /g | /g | Einzelwerte | Mittelwert |
| 1 | 0,320 | 0,698 | 0,378 | 118 % | 125 % |
| | 0,310 | 0,709 | 0,399 | 129 % | |
| | 0,320 | 0,726 | 0,406 | 127 % | |
| 2 | 0,410 | 1,054 | 0,644 | 157 % | 158% |
| | 0,500 | 1,283 | 0,783 | 157 % | |
| | 0,430 | 1,115 | 0,685 | 159 % | |
| 3 | 0,190 | 0,224 | 0,034 | 18 % | 15% |
| | 0,194 | 0,219 | 0,025 | 13 % | |
| | 0,200 | 0,226 | 0,252 | 13 % | |
| 4 | 0,243 | 0,330 | 0,087 | 36 % | 36% |
| | 0,231 | 0,320 | 0,088 | 38 % | |
| | 0,235 | 0,315 | 0,080 | 34 % | |

**Tabelle 2: Testung auf 35 %-ige Gelatine-Lösung**

| Probe | Einwaage | Auswaage nach 24 Std. | Wasseraufnahme nach 24 Std. | Wasseraufnahme nach 24 Std. (bezogen auf Einwaage) | |
|---|---|---|---|---|---|
| | / g | / g | / g | Einzelwerte | Mittelwert |
| 1 | 0,308 | 0,577 | 0,269 | 87 % | 91 % |
| | 0,318 | 0,575 | 0,257 | 81 %^{#} | |
| | 0,313 | 0,615 | 0,302 | 96 % | |
| 2 | 0,496 | 1,069 | 0,573 | 116 % | 115 % |
| | 0,441 | 0,950 | 0,509 | 115 % | |
| | 0,449 | 0,965 | 0,516 | 115 % | |
| 3 | 0,189 | 0,226 | 0,037 | 19 % | 18 % |
| | 0,190 | 0,225 | 0,035 | 18 % | |
| | 0,192 | 0,227 | 0,035 | 18 % | |
| 4 | 0,227 | 0,281 | 0,054 | 24 % | 25% |
| | 0,237 | 0,296 | 0,059 | 25 % | |
| | 0,238 | 0,297 | 0,059 | 25 % | |

| | | | | | |
|---|---|---|---|---|---|
| # Teststück lag nicht vollflächig auf der Gelatineoberfläche (nicht berücksichtigt) | | | | | |

Die mit Tabelle 1 wiedergegeben Ergebnisse stellen eine Näherung für das Verhalten der Wundkontaktschichten auf mäßig bis stark exsudierenden Wunden dar. Demnach nimmt eine Wundkontaktschicht gemäß Beispiel 5 (Probe 2) in 24 Std. im Mittel 158 % des Eigengewichtes an Flüssigkeit auf. Im Vergleich hierzu zeigt ein auf dem Markt bereits erhältliches Produkt eine viel geringere Feuchtigkeitsaufnahme. Die Werte liegen im Mittel für Probe 4 bei 36 % und bei Probe 3 bei 15 %. Einen ähnlichen Trend kann mit den Ergebnissen in Tabelle 2 gezeigt werden. Diese Untersuchung stellt eine Simulation an einer schwach bis mäßig exsudierende Wunde dar. Demnach nimmt eine Wundkontaktschicht gemäß Beispiel 5 (Probe 2) in 24 Std. im Mittel 115 % des Eigengewichtes an Flüssigkeit auf. Im Vergleich hierzu zeigen die auf dem Markt befindlichen Produkte eine geringere Feuchtigkeitsaufnahmekapazität. Die Werte liegen im Mittel für Probe 4 bei 25 % und bei Probe 3 bei 19 %. Werden die Ergebnisse der beiden Testungen miteinander verglichen, so lässt sich darüber hinaus feststellen, dass eine vorliegende Wundkontaktschicht auch bedarfsgerechte Feuchtigkeitsaufnahme zeigt, dass heißt, das bei stark exsudierenden Wunden mehr Feuchtigkeit aufgenommen wird als bei eher mäßig exsudierenden Wunden.

### 6) Wundauflage 1

Mit Figur 2 ist eine erfindungsgemäße Wundauflage (20) als so genanntes Inseldressing wiedergegeben. Die Wundauflage besteht aus einer Abdeckschicht (24), und einer Wundkontaktschicht (21). Die Wundkontaktschicht ihrerseits besteht aus einem Trägermaterial (22), das ein hydrophobes Nonwoven aus Polyesterfasern ist, das mit einer Zusammensetzung (23) laut Beispiel 1 bestrichen ist, wobei ein Teil der Polyesterfasern mit elementarem Silber beschichtet sind. Die Zusammensetzung überdeckt mit einer Auftragsmenge von 240 g/ m² vollständig das Polyester-Nonwoven (wasserstrahlverfestigt, Flächengewicht 80 g/ m²). Die Wundkontaktschicht ist mit einer Abdeckschicht (24) überdeckt, die mit einem Polyacrylat-Haftklebemittel (25) vollflächigen beschichtet ist. Die Abdeckschicht ist ein 30 µm dicker Polyurethanfilm mit einer Wasserdampfdurchlässigkeit von 1100 g/ m² / 24 Std., die allseitig über die Umfangsgrenzen der Wundkontaktschicht hinaus reicht, so dass mittels der klebenden Ränder der Abdeckschicht (26a, 26b) die Wundkontaktschicht auf der Haut eines Patienten befestigt werden kann. Zugleich ist die Wundkontaktschicht (22) mittels des Haftklebers (25) auf der Abdeckschicht fixiert.

### 7) Wundauflage 2

Mit Figur 3 ist eine weitere erfindungsgemäße Wundauflage (30) gezeigt, die gegenüber der mit Figur 2 gezeigten Wundauflage (20) eine zusätzliche absorbierende Schicht (37) aufweist. Diese zusätzliche absorbierende Schicht (37) besteht aus einem hydrophilen, offenzelligen Polyurethanschaum mit einem Flächengewicht von 500 g/ m² bei einer Dicke von 5 mm. Die absorbierende Schicht ist mittels der Haftklebeschicht (35) aus einem Acrylat-Dispersionshaftkleber auf der Abdeckschicht fixiert. Die Abdeckschicht besteht aus einem Polyurethanfilm mit einer Dicke von 25 µm und einer Wasserdampfdurchlässigkeit von 1200 g/ m²/ 24 Std.. Die Wundkontaktschicht (31) besteht einem Trägermaterial (32), das auf der wundzugewandten Seite bzw. Oberflächen mit einer Zusammensetzung laut Beispiel 2 bestrichen ist (180 g/ m²). Das Trägermaterial (1) besteht aus einem mit elementarem Silber beschichteten Polyamidträgergewirk, das aus Nylon 6 Trilobalfäden mit 50 µm Kantenlänge hergestellt ist. Das Rohmaterial (ohne Versilberung) hat ein Flächengewicht von 25 bis 30 g/m² und eine Fadenfeinheit von dtex= 22/1. In dem Material sind auf einem Quadratmeter zwischen 11.360 und 13.640 m Faden verwirkt. Dies bedeutet, dass die Oberfläche zwischen 1,7 m² und 2,04 m² liegt. Die Schichtdicke des Silbers auf dem Trägermaterial beträgt ca. 700 nm. Zwischen versilbertem und unversilbertem Produkt ist mit den herkömmlichen Mitteln (Lichtmikroskop) kein Unterschied in der Dreiecksseitenlänge feststellbar. Das Flächengewicht des versilberten Gewirks beläuft sich auf 35 +/- 3 g/m². Dabei beträgt der Gehalt an elementarem Silber ca. 130 mg/ g Polyamidträgergewirk. Derartige silberbeschichtete Gewirke werden von der Firma Statex, Bremen unter dem Namen Shieldex® Tüll Typ Charmeuse vertrieben und wurden hier als Trägermaterial eingesetzt. Die Wundauflage ist besonders zum Einsatz bei stark nässenden Wunden einzusetzen. Die Wundauflage pflegt durch den Anteil an Triglyceriden in der Zusammensetzung die periphere Wundhaut und zeigt auch bei längerem Gebrauch keine Wundverklebung.

## Patentansprüche

1. Wundkontaktschicht (10, 21, 31) umfassend
a) eine Zusammensetzung zur Wundbehandlung (2a, 2b) mit weniger als 10 Gew.-% Wasser umfassend 60 bis 95 Gew.-% hydrophile Basis, in die 5 bis 40 Gew.-% Hydrocolloide dispergiert sind, wobei die hydrophile Basis 0,5 bis 50 Gew.-% mindestens eines Emulgators umfasst und
b) ein ein antimikrobiell wirkendes Metall umfassendes Trägermaterial (1) für die Zusammensetzung,
**dadurch gekennzeichnet, dass**
- die hydrophile Basis weiterhin 20 bis 80 Gew.-% Mono-, Di- und/ oder Triglyceriden und/ oder Voll- oder Partialester Oligomerer des Glycerins umfasst,
- die hydrophile Basis weiterhin 10 bis 30 Gew.-% unpolare Lipide umfasst.

2. Wundkontaktschicht (10, 21, 31) nach Anspruch 1 **dadurch gekennzeichnet, dass** die hydrophile Basis weniger als 10 Gew.-% Wasser umfasst oder wasserfrei ist.

3. Wundkontaktschicht (10, 21, 31) nach mindestens einem der vorgenannten Ansprüche **dadurch gekennzeichnet, dass** die hydrophile Basis eine Creme, Cremegrundlage oder Salbe ist.

4. Wundkontaktschicht (10, 21, 31) nach mindestens einem der vorgenannten Ansprüche **dadurch gekennzeichnet, dass** die Hydrocolloide in Partikelform vorliegen und die Partikel einen Wassergehalt von weniger als 10 Gew.-% bezogen auf das Gewicht der Hydrocolloidpartikel aufweisen.

5. Wundkontaktschicht (10, 21, 31) nach mindestens einem der vorgenannten Ansprüche **dadurch gekennzeichnet, dass** die Hydrocolloide ausgewählt werden aus der Gruppe der Cellulose oder Derivate oder deren Salze sowie Alginsäure oder deren Derivate oder Salze.

6. Wundkontaktschicht (10, 21, 31) nach mindestens einem der vorgenannten Ansprüche **dadurch gekennzeichnet, dass** es sich bei den unpolaren Lipiden um Lipide aus der Gruppe Vaseline, Petrolatum, Parafinöl oder Waxe handelt.

7. Wundkontaktschicht (10, 21, 31) nach mindestens einem der vorgenannten Ansprüche **dadurch gekennzeichnet, dass** der Emulgator ein ionischer O/W-Emulgator ist.

8. Wundkontaktschicht (10, 21, 31) nach mindestens einem der vorgenannten Ansprüche **dadurch gekennzeichnet, dass** der Emulgator ein nichtionischer W/O-Emulgator ist.

9. Wundkontaktschicht (10, 21, 31) nach mindestens einem der vorgenannten Ansprüche **dadurch gekennzeichnet, dass** das Trägermaterial (1) ein Nonwoven, Gestrick, Gewirk oder Gewebe ist.

10. Wundkontaktschicht (10, 21, 31) nach mindestens einem der vorgenannten Ansprüche **dadurch gekennzeichnet, dass** das Metall als Beschichtung auf das Trägermaterial (1) aufgebracht oder auf bzw. in das Trägermaterial imprägniert oder inkorporiert ist.

11. Wundkontaktschicht (10, 21, 31) nach mindestens einem der vorgenannten Ansprüche **dadurch gekennzeichnet, dass** das Trägermaterial ein Nonwoven oder ein textiles Material wie Gestrick, Gewirk oder Gewebe ist, das mit einem antimikrobiell wirkenden Metall beschichtet ist.

12. Wundkontaktschicht (10, 21, 31) nach mindestens einem der vorgenannten Ansprüche **dadurch gekennzeichnet, dass** das Trägermaterial (1) ein Nonwoven ist, das aus einem Fasergemisch aus antimikrobiell wirkendem Metall umfassenden Fasern und metallfreien Fasern besteht.

13. Wundkontaktschicht (10, 21, 31) nach mindestens einem der vorgenannten Ansprüche **dadurch gekennzeichnet, dass** das Trägermaterial (1) aus einem hydrophoben Gestrick, Gewirk oder Gewebe besteht.

14. Wundkontaktschicht (10, 21, 31) nach mindestens einem der vorgenannten Ansprüche **dadurch gekennzeichnet, dass** das Trägermaterial (1) ein Polyamid-Gewirk umfasst.

15. Wundkontaktschicht (10, 21, 31) nach mindestens einem der vorgenannten Ansprüche **dadurch gekennzeichnet, dass** das antimikrobielle Metall elementares Silber, ein Silbersalz oder ein Silberkomplex ist.

16. Wundauflage (20, 30) umfassend eine Wundkontaktschicht (10, 21, 31) nach mindestens einem der Ansprüche 1 - 15 und eine Abdeckschicht (24, 34).

17. Wundauflage (20, 20) gemäß Anspruch 16 **dadurch gekennzeichnet, dass** die Wundauflage weiterhin eine absorbierende Schicht (37) umfasst, die der Wundkontaktschicht (10, 21, 31) benachbart ist.

## Claims

1. Wound contact layer (10, 21, 31) comprising
a) a composition for wound treatment (2a, 2b) with less than 10% by weight of water comprising 60 to 95% by weight of hydrophilic base in which 5 to 40% by weight of hydrocolloids are dispersed, where the hydrophilic base comprises 0.5 to 50% by weight of at least one emulsifier and
b) a carrier material (1) comprising an antimicrobial metal for the composition,
**characterized in that**
- the hydrophilic base further comprises 20 to 80% by weight of mono-, di- and/or triglycerides and/or full or partial ester oligomers of glycerol,
- the hydrophilic base further comprises 10 to 30% by weight of nonpolar lipids.

2. Wound contact layer (10, 21, 31) according to Claim 1, **characterized in that** the hydrophilic base comprises less than 10% by weight of water or is free from water.

3. Wound contact layer (10, 21, 31) according to at least one of the preceding claims, **characterized in that** the hydrophilic base is a cream, cream base or ointment.

4. Wound contact layer (10, 21, 31) according to at least one of the preceding claims, **characterized in that** the hydrocolloids are present in particle form and the particles have a water content of less than 10% by weight, based on the weight of the hydrocolloid particles.

5. Wound contact layer (10, 21, 31) according to at least one of the preceding claims, **characterized in that** the hydrocolloids are selected from the group of cellulose or derivatives or salts thereof, and alginic acid or derivatives or salts thereof.

6. Wound contact layer (10, 21, 31) according to at least one of the preceding claims, **characterized in that** the nonpolar lipids are lipids from the group Vaseline, petrolatum, paraffin oil or waxes.

7. Wound contact layer (10, 21, 31) according to at least one of the preceding claims, **characterized in that** the emulsifier is an ionic O/W emulsifier.

8. Wound contact layer (10, 21, 31) according to at least one of the preceding claims, **characterized in that** the emulsifier is a nonionic W/O emulsifier.

9. Wound contact layer (10, 21, 31) according to at least one of the preceding claims, **characterized in that** the carrier material (1) is a nonwoven, weft-knitted fabric, warp-knitted fabric or woven.

10. Wound contact layer (10, 21, 31) according to at least one of the preceding claims, **characterized in that** the metal is applied as coating to the carrier material (1) or is impregnated or incorporated on and/or into the carrier material.

11. Wound contact layer (10, 21, 31) according to at least one of the preceding claims, **characterized in that** the carrier material is a nonwoven or a textile material such as weft-knitted fabric, warp-knitted fabric or woven which is coated with an antimicrobial metal.

12. Wound contact layer (10, 21, 31) according to at least one of the preceding claims, **characterized in that** the carrier material (1) is a nonwoven which consists of a fibre mixture of fibres comprising antimicrobial metal and metal-free fibres.

13. Wound contact layer (10, 21, 31) according to at least one of the preceding claims, **characterized in that** the carrier material (1) consists of a hydrophobic weft-knitted fabric, warp-knitted fabric or woven.

14. Wound contact layer (10, 21, 31) according to at least one of the preceding claims, **characterized in that** the carrier material (1) comprises a polyamide warp-knitted fabric.

15. Wound contact layer (10, 21, 31) according to at least one of the preceding claims, **characterized in that** the antimicrobial metal is elemental silver, a silver salt or a silver complex.

16. Wound covering (20, 30) comprising a wound contact layer (10, 21, 31) according to at least one of Claims 1-15 and a covering layer (24, 34).

17. Wound covering (20, 30) according to Claim 16, **characterized in that** the wound covering further comprises an absorbent layer (37) which is adjacent to the wound contact layer (10, 21, 31).

## Revendications

1. Couche de contact avec une plaie (10, 21, 31), comprenant
a) une composition pour le traitement de la plaie (2a, 2b) avec moins de 10 % en poids d'eau comprenant 60 à 95 % en poids d'une base hydrophile, dans laquelle 5 à 40 % en poids d'hydrocolloïdes sont dispersés, dans laquelle la base hydrophile comprend 0,5 à 50 % en poids d'au moins un émulsifiant, et
b) un matériau de support (1) comprenant un métal ayant une action antimicrobienne pour la composition,
**caractérisée en ce que**
- la base hydrophile comprend en outre 20 à 80 % en poids de mono, di- ou triglycérides et/ou des esters complets ou partiels d'oligomères de la glycérine,
- la base hydrophile comprend en outre 10 à 30 % en poids de lipides non polaires.

2. Couche de contact avec une plaie (10, 21, 31) selon la revendication 1, **caractérisée en ce que** la base hydrophile comprend moins de 10 % en poids d'eau ou est exempte d'eau.

3. Couche de contact avec une plaie (10, 21, 31) selon au moins une des revendications précédentes, **caractérisée en ce que** la base hydrophile est une crème, une base de crème ou une pommade.

4. Couche de contact avec une plaie (10, 21, 31) selon au moins une des revendications précédentes, **caractérisée en ce que** les hydrocolloïdes sont présents sous forme de particules et les particules présentent une teneur en eau de moins de 10 % en poids, rapportée au poids des particules d'hydrocolloïdes.

5. Couche de contact avec une plaie (10, 21, 31) selon au moins une des revendications précédentes, **caractérisée en ce que** les hydrocolloïdes sont choisis dans le groupe des celluloses ou de leurs dérivés ou de leurs sels ainsi que l'acide alginique ou ses dérivés ou ses sels.

6. Couche de contact avec une plaie (10, 21, 31) selon au moins une des revendications précédentes, **caractérisée en ce que** les lipides non polaires sont des lipides du groupe comprenant la vaseline, le pétrolatum, l'huile de paraffine ou les cires.

7. Couche de contact avec une plaie (10, 21, 31) selon au moins une des revendications précédentes, **caractérisée en ce que** l'émulsifiant est un émulsifiant ionique huile/eau.

8. Couche de contact avec une plaie (10, 21, 31) selon au moins une des revendications précédentes, **caractérisée en ce que** l'émulsifiant est un émulsifiant non ionique eau/huile.

9. Couche de contact avec une plaie (10, 21, 31) selon au moins une des revendications précédentes, **caractérisée en ce que** le matériau de support (1) est un non-tissé, un article tricoté, un article à mailles ou un tissu.

10. Couche de contact avec une plaie (10, 21, 31) selon au moins une des revendications précédentes, **caractérisée en ce que** le métal est déposé sous forme de revêtement sur le matériau de support (1) ou est imprégné ou incorporé sur ou dans le matériau de support (1).

11. Couche de contact avec une plaie (10, 21, 31) selon au moins une des revendications précédentes, **caractérisée en ce que** le matériau de support est un non-tissé ou un matériau textile comme un tricot, un article à mailles ou un tissu, qui est revêtu avec un métal ayant une action antimicrobienne.

12. Couche de contact avec une plaie (10, 21, 31) selon au moins une des revendications précédentes, **caractérisée en ce que** le matériau de support (1) est un non-tissé, qui se compose d'un mélange de fibres en fibres comprenant un métal ayant une action antimicrobienne et en fibres sans métal.

13. Couche de contact avec une plaie (10, 21, 31) selon au moins une des revendications précédentes, **caractérisée en ce que** le matériau de support (1) se compose d'un tricot, d'un article à mailles ou d'un tissu hydrophobe.

14. Couche de contact avec une plaie (10, 21, 31) selon au moins une des revendications précédentes, **caractérisée en ce que** le matériau de support (1) comprend un tricot en polyamide.

15. Couche de contact avec une plaie (10, 21, 31) selon au moins une des revendications précédentes, **caractérisée en ce que** le métal antimicrobien est l'argent élémentaire, un sel d'argent ou un complexe d'argent.

16. Pansement (20, 30) comprenant une couche de contact avec une plaie (10, 21, 31) selon au moins une des revendications 1 à 15 et une couche de recouvrement (24, 34).

17. Pansement (20, 30) selon la revendication 16, **caractérisé en ce que** le pansement comprend en outre une couche absorbante (37), qui est voisine de la couche de contact avec une plaie (10, 21, 31).
